# EUROPÄISCHE PATENTSCHRIFT

(11) **EP 1 960 326 B1**
(45) Veröffentlichungstag und Bekanntmachung des Hinweises auf die Patenterteilung: **21.09.2016**
(21) Anmeldenummer: 06818978.6
(22) Anmeldetag: 05.12.2006
(51) Int. Cl.: C07C 1/20, A61K 8/31

(54) **VERFAHREN ZUR HERSTELLUNG VON KOHLENWASSERSTOFFEN**
PROCESS FOR THE PRODUCTION OF HYDROCARBONS
PROCÉDÉ POUR LA PRODUCTION D'HYDROCARBURES

(30) Priorität: 14.12.2005 EP 05027253
(43) Veröffentlichungstag der Anmeldung: 27.08.2008
(73) Patentinhaber: Cognis IP Management GmbH, 40589 Dusseldorf (DE)
(72) Erfinder: FALKOWSKI, Jürgen, 40789 Monheim (DE); DIERKER, Markus, 40597 Düsseldorf (DE); NEUSS, Michael, 50997 Köln (DE); SCHMID, Karl Heinz, 40822 Mettmann (DE); WÜRKERT, Stephan, 80639 München (DE); ZANDER, Lars, 41569 Rommerskirchen (DE)
(74) Vertreter: BASF IP Association
(86) Internationale Anmeldenummer: PCT/EP2006/011647
(87) Internationale Veröffentlichungsnummer: WO 2007/068371

(56) Entgegenhaltungen:
- GB-A- 543 327
- ELMER J BADIN: "Catalytic dehydrogenation. I. Catalytic conversion of alcohols into aldehydes, paraffins and olefins" JOURNAL OF THE AMERICAN CHEMICAL SOCIETY., Bd. 65, Nr. 10, 1943, Seiten 1809-1813, XP002382112 XXXX

## Beschreibung

Die vorliegende Erfindung betrifft ein Verfahren zur Herstellung von Kohlenwasserstoffen aus Fettalkoholen und beschreibt die Verwendung derartiger Kohlenwasserstoffen, vorzugsweise in kosmetischen Mitteln.

Leichtflüchtige Ölkörper, so genannte light emollients', werden von der kosmetischen Industrie in einer Vielzahl von Formulierungen verwendet. Insbesondere für die dekorative Kosmetik bzw. in pflegenden Formulierungen werden große Mengen an leichterflüchtigen Komponenten eingesetzt. Bei diesen Komponenten kann es sich beispielsweise um flüchtige, cyclische Silikone (z.B: Cyclopentasiloxan oder Cyclomethicon) oder Kohlenwasserstoffe aus petrochemischen Prozessen handeln. Bei den zuletzt genannten Stoffen handelt es sich aufgrund ihrer Herstellung überwiegend um Gemische aus linearen und verzweigten Kohlenwasserstoffen, deren Flammpunkt durchaus unter 50 °C (wie z.B. beim Isododecan) liegen kann. Beispiele und anwendungstechnische Beschreibungen derartiger Formulierungen können in Standardwerken, wie zum Beispiel: 'Handbook of Cosmetic Science and Technology', A. Barel, M. Paye, H. Maibach, Marcel Dekker Inc. 2001 nachgelesen werden. Aus toxikologischen bzw. sicherheitstechnischen Gründen besteht jedoch in Zukunft Bedarf nach alternativen Rohstoffen für derartige Formulierungsaufgaben.

Die GB 543,327 beschreibt die Herstellung von langkettigen aliphatischen Kohlenwasserstoffen, die mindestens acht Kohlenstoffatome enthalten, aus langkettigen Aldehyden oder Alkoholen. Bei der Umsetzung des Alkohols in Gegenwart von Wasserstoff und Nickel-Katalysator werden 10 % Katalysator, bezogen auf den eingesetzten Fettalkohol, eingesetzt.

Die Aufgabe der Erfindung bestand darin, alternative Rohstoffe zu finden, die einerseits ökologisch bzw. toxikologisch unbedenklich sind und andererseits in typischen kosmetischen Formulierungen ohne anwendungstechnische Einschränkungen direkt ausgetauscht werden können.

Eine weitere Aufgabe der Erfindung besteht darin, bei der reduktiven Dehydroxymethylierung von primären Alkoholen möglichst wenig Mengen an Katalysator einzusetzen.

Es wurde nun gefunden, dass über eine speziell geführte katalysatorarme Reaktion Hydroxylverbindungen, wie z.B. Fettalkohole, mit hoher Selektivität in reine Kohlenwasserstoffe mit um ein C-Atom verkürzter Kettenlänge überführt werden können.

Ein erster Gegenstand der vorliegenden Erfindung betrifft daher ein Verfahren zur Herstellung von linearen gesättigten Alkanen aus primären Alkoholen, deren C-Kette ein Kohlenstoffatom mehr enthält als das Alkan, durch reduktive Dehydroxymethylierung der primären Alkohole in Gegenwart von Wasserstoff und einem Nickel-haltigen Katalysator bei Temperaturen von 100 bis 300°C und Drücken von 2 bis 250 bar, wobei während der Reaktion entstehendes Wasser entfernt wird, das dadurch gekennzeichnet ist, dass als primäre Alkohole Fettalkohole mit 8 bis 24 Kohlenstoffatomen ausgewählt sind und der Katalysator in Mengen von 0,1 bis 3 Gew.-% zugesetzt wird.

Die hierbei ablaufende Reaktion wird als Dehydroxymethylierung oder auch reduktive Dehydroxymethylierung bezeichnet. Diese Reaktion ist für organische primäre Alkohole an sich bekannt. Aus dem Aufsatz "Hydrogenolysis of Alcohols"; Autoren: Hermann Pines und T.P. Kobylinski, Journal of Catalysis 17, 375-383 (1970) ist bekannt, dass Neopentylakohol u.a. zu Isobutan umgesetzt werden kann. Auch wird die Reaktion von Butanol zu Propan in Gegenwart von Nickel-Katalysatoren in einer Wasserstoffatmosphäre beschrieben. Es wird aber nicht die Verwendung langkettiger Fettalkohole für solche Reaktionen genannt. Die in diesem Aufsatz beschriebenen Reaktionen finden auch nicht in technischen Maßstab statt sondern wurden im Mikromaßstab durchgeführt. In dem Aufsatz "Direct Reduction of Alcohols to Hydrocarbons"; Autoren: W.F. Maier, I. This, P. Schleyer. Zeitschrift für Naturforschung, Teil B, 1982, 37B(3) wird zwar ebenfalls die reduktive Demethylierung primärer organischer Alkohole beschrieben, es werden aber keine langkettigen Fettalkohole als geeignete Edukte offenbart oder nahe gelegt. Die UK 1,051,826 beschreibt die reduktive Demethylierung von Diolen mit Nickel-Katalysatoren in einer Wasserstoffatmosphäre. Die katalytische Dehydrogenierung von Fettalkoholen ist beschrieben bei Elmer J. Badin, Catalytic dehydrogenation I. Catalytic conversion of alcohols into aldehydes, paraffins and olefins, im Journal of the American Chemical Society. Bd. 65. Nr. 10, 1943, S. 1809-1813.

Das dort durchgeführte Verfahren findet bei Normaldruck statt und man erhält nur geringe Ausbeuten an Paraffinen.

Aus den erfindungsgemäß erhaltenen Reaktionsgemischen können, vorzugsweise nach Aufreinigung der Rohprodukte, z.B. nach fraktionierter Destillation und weiterhin vorzugsweise nachfolgender Desodorierung, hochreine Kohlenwasserstoffe mit definierter Kettenlänge gewonnen werden. Die so erhaltenen Kohlenwasserstoffe definierter Kettenlänge können entweder als Einzelkomponenten in kosmetischen Formulierungen als sog. 'light emollients' verwendet werden oder definiert gemischt werden, um spezielle Eigenschaften, wie z.B. Spreitverhalten, Flüchtigkeit oder auch einen Flammpunkt einstellen zu können.

Die hydroxylhaltige Komponenten können Fettalkohole der oben angegebenen Kettenlänge sein, die wiederum nach einem dem Fachmann bekannten Verfahren aus nachwachsenden Rohstoffen wie z.B. Kokos-, Palm- oder Palmkernöl über Umesterung mit Methanol und anschließender Hydrierung hergestellt werden. Neben reinen Fettalkoholen können prinzipiell und bevorzugt auch andere, technisch hergestellte, lineare oder verzweigte, einoder mehrwertige Alkohole, Alkoholgemische oder derivatisierte Alkohole eingesetzt werden. Besonders bevorzugt ist die Verwendung von Fettalkoholen mit geradzahligen C-Ketten, da so leicht die ansonsten schwer zugänglichen ungeradzahligen Alkane erhalten werden können. Bevorzugt werden als primäre Alkohole solche der allgemeinen Formel R-OH ausgewählt, in der R für einen gesättigten, linearen Alkylrest mit 8 bis 18, vorzugsweise 10 bis 16 und insbesondere 12 bis 16 Kohlenstoffatomen steht.

Die Reaktion der Alkohole zu den Kohlenwasserstoffen muss unter Zugabe von Wasserstoff und unter gleichzeitiger Wasserentfernung erfolgen.

Als Katalysatoren sind Nickel-haltige Katalysatoren besonders geeignet. Bevorzugte Nickel-Katalysatoren sind handelsübliche Ni-haltige Hydrierkatalysatoren, wie z.B. Katalysatoren der Fa. Engelhard oder Kata Leuna. Die Katalysatoren können hierbei sowohl als Suspensionskatalysatoren für ein Semi-Batch - Verfahren, als auch als Festbettkatalysatoren für ein kontinuierliches Verfahren eingesetzt werden. Die Katalysatoren sind erfindungsgemäß im Mengen von 0, 1 bis 3 Gew.-%, bezogen auf die Menge an primären Fettalkoholen in der Reaktionsmischung vorhanden. Bevorzugt können auch Katalysatoren in Mengen von 0,2 bis 2 Gew.-% und insbesondere in Mengen von 0,5 bis 1,0 Gew.-% Verwendung finden. Bei einem Suspensionsverfahren hat sich eine Katalysatorkonzentration von 0,1 - 2 Gew.-% bezüglich auf die eingesetzte Menge an Fettalkohol als geeignet erwiesen, wobei der bevorzugte Bereich bei 0,5 - 1,0 Gew.-% Ni liegt.

Die für das Verfahren erforderliche Reaktionstemperatur beträgt 100 °C - 300°C, wobei der bevorzugte Bereich bei 200 - 280°C liegt und insbesondere bevorzugt bei 220°C - 260°C liegt.

Der für das Verfahren geeignete Reaktionsdruck beträgt 2 - 300 bar, wobei 2 bis 250 bar, und insbesondere 5 bis 100 bar bevorzugt ist. Besonders bevorzugt ist der Bereich von 5 - 80 bar und insbesondere von 10 - 50 bar.

Während der Reaktion entstehendes Reaktionswasser ist zu entfernen. Daher hat es sich als vorteilhaft erwiesen, Wasserstoff zu dem mit suspendiertem Katalysator vorgelegtem Alkohol zu dosieren und gleichzeitig entstehendes Reaktionswasser bzw. Reaktionsgase aus dem Reaktor zu entfernen. Bei einem kontinuierlichen Verfahren kann diese Wasserauskreisauskreisung beispielsweise in einem mehrstufigen Verfahren erfolgen. Das entstandene Reaktionsgemisch muss anschließend zur Katalysatorabtrennung filtriert werden. Danach wird zur Entfernung des Restalkoholgehaltes bzw. von Spuren dimerer Reaktionsprodukte eine fraktionierte Destillation durchgeführt. Das hierbei erhaltene Sumpfprodukt kann für den nächsten Ansatz recyclisiert werden.
Anschließend kann zur Geruchsverbesserung noch eine Desodorierung nachgeschaltet werden.
Die vorliegende Erfindung beschreibt auch die Verwendung der gemäß dem obigen Verfahren hergestellten Kohlenwasserstoffe in kosmetischen Mitteln.

Insbesondere ungradzahlige Kohlenwasserstoffe sind nach dem erfindungsgemäßen Verfahren leicht zugänglich. Daher betrifft die vorliegende Erfindung insbesondere die Verwendung von linearen, gesättigten ungradzahligen Alkanen mit einer C Zahl von 7 bis 23 in kosmetischen Mitteln. Exemplarisch seien genannt n- Nonane, n-Undecan und n-Tridecan, n-Heptadecan.

Die vorliegende Erfindung beschreibt die Verwendung einzelner nach dem erfindungsgemäßen Verfahren hergestellter Kohlenwasserstoffe sowie beliebiger Mischung verschiedener Kohlenwasserstoffe.

Die erfindungsgemäß hergestellten Kohlenwasserstoffe können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung als Ölkörper.

Mit der hier getätigten Erfindung besteht gezielt die Möglichkeit, Kohlenwasserstoffe definierter Kettenlänge als Einzelkomponenten in kosmetischen Formulierungen als sog. ,light emollients' zu verwenden oder sogar definiert zu mischen, um spezielle Eigenschaften, wie z.B. Spreitverhalten, Flüchtigkeit oder auch Flammpunkte einstellen zu können. Insbesondere durch die Möglichkeit einer Mischung dieser Kohlenwasserstoffe nach dem Baukastenprinzip ergeben sich somit wesentliche Vorteile gegenüber Kohlenwasserstoffen aus petrochemischen Quellen, die fast ausschließlich als komplexe Gemische aus verzweigten und unverzweigten Kohlenwasserstoffen vorliegen. Eine weitere destillative Aufarbeitung ist in diesen Fällen nur mit hohem Aufwand möglich, bzw. hätte das Problem, das Restmengen an unverwünschten Isomeren im Produkt verbleiben würden. Darüber hinaus ist - eine insbesondere für kosmetische Anwendungen wichtige - toxikologische Bewertung eines definierten Kohlenwasserstoffes bzw. einer definierten Kohlenwasserstoffinischung sehr viel einfacher und sicherer.

### Beispiele

### 1) Herstellung von Tridecan aus 1-Tetradecanol

1000 g 1-Tetradecanol (4,7 mol; Lorol C 14 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240 °C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

Eine GC-Analyse ergibt folgende Zusammensetzung:
89,0 % Tridecan
2,1 % Tetradecan
4,1 % 1-Tetradecanol
4,2 % Dimere Reaktionsprodukte

Dieses Reaktionsprodukt wird danach in einer Destillation zum reinen Tridecan fraktioniert und anschließend mit Stickstoff desodoriert. Man erhält ein farbloses, dünnflüssiges und geruchsarmes Produkt.

### 2) Herstellung von Undecan aus 1-Dodecanol

1000 g 1-Dodecanol (5,4 mol; Lorol C 12 der Fa. Cognis) wurden in einem rührbaren Druckbehälter mit 10 g eines Nickelkatalysators (Ni-5249 P der Fa. Engelhard; Ni-Gehalt = 63 Gew%) vorgelegt und auf 240°C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

Eine GC-Analyse ergibt folgende Zusammensetzung:
68,4 % Undecan
0,6 % Dodecan
21,7% 1-Dodecanol
7,2 % Dimere Reaktionsprodukte

Dieses Reaktionsprodukt wurde danach Destilliert, um das Undecan rein zu erhalten. Dieses wurde anschließend mit Stickstoff desodoriert. Man erhält ein farbloses, dünnflüssiges und geruchsarmes Produkt.

### 3) Kosmetische Zubereitungen, enthaltend Undecan bzw. Tridecan

Die im Folgenden genannten Beispiele enthalten entweder Undecan (erhalten gemäß Beispiel 2) oder Tridecan (erhalten nach Beispiel 1).

**Beispiel 3.1 Allzweck Creme**

| Phase | Komponente | INCI | Gew.% |
|---|---|---|---|
| | Handelsname | | |
| I. | DEHYMULS® E | Dicocoyl Pentaerhytithyl | |
| | | Distearyl Citrate (and) | |
| | | Sorbitan Sesquioleate (and) | |
| | | Cera Alba (Beeswax) (and) | |
| | | Aluminium Stearates | 3,00 |
| | DEHYMULS® | Polyglyceryl 2 | |
| | PGPH | Dipolyhydroxystearate | 2,00 |
| | CETIOL® OE | Dicaprylyl Ether | 3,00 |
| | CETIOL® 868 | Ethylhexyl Stearate | 4,00 |
| | MYRITOL® 331 | Cocoglycerides | 2,00 |
| | Undecane/Tridecane | | 6,00 |
| II. | Glycerin 86% | | 5,00 |
| | MgSO₄ x 7H₂O | | 1,00 |
| | Wasser, deionisiert | | 74,00 |
| III. | Konservierungsmittel | | q.s. |

### Herstellung:

Die Komponenten der Phase I wurden bei 80 bis 85 °C geschmolzen und zur Homogenität verrührt. Die Komponenten der Phase II wurden auf 80 bis 85 °C erhitzt und langsam, unter Rühren zur Phase I zugegeben. Es wurde für weitere 5 Minuten bei dieser Temperatur gerührt. Danach wurde die Emulsion unter Rühren abgekühlt und bei 65 bis 55 °C homogenisiert. Sobald die Emulsion homogen erscheint, wurde unter Rühren weiter auf 30 °C abgekühlt. Danach wurden Komponenten der Phase III zugegeben und erneut gerührt.

**Beispiel 3.2. Balsam zur Befeuchtung und zum Schutz der Lippen**

| Phase | Komponente | INCI | Gew.-% |
|---|---|---|---|
| | Handelsname | | |
| I. | Cerilla Raffinee G* | Candelilla(Euphorbia Cerifera) Wax | 7,53 |
| | CUTINA® LM conc. | Polyglyceryl-2 Dipolyhydroxystearate | |
| | | and Octyldodecanol and Copernicia | |
| | | Cerifera (Carnauba) Wax and Euphorbia | |
| | | Cerifera (Candelilla) Wax and Beeswax | |
| | | and Cetearyl Glucoside and Cetearyl | |
| | | Alcohol | 6,57 |
| | Paracera M (Paramelt) | Mycrocrystalline Wax | 2,45 |
| | Cerewax M85/C(SCLR) | Ceresin | 2,08 |
| | Colophane claire type Y | Rosin | 1,89 |
| | Cerauba T1* | Carnauba (Copernica Cerifera)Wax | 1,86 |
| | Cerabeil blanche 1* | Beeswax | 0.78 |
| | Undecane/Tridecane | | 15.57 |
| | EUTANOL® G | Octyldodecanol | 14.87 |
| | Vaseline F7850(Fina) | Petrolatum | 6,84 |
| | Crodamol ML(Croda) | Myristyl Lactate | 1,13 |
| | ELESTAB®366 | | 0.43 |
| II. | Castor oil | Castor Oil | 35.00 |
| III. | IRWINOL® LS 9319 | African wild mango butter | 3.00 |

| | | | |
|---|---|---|---|
| * erhältlich von Lambert- Riviere (France) | | | |

Herstellung: Phase I wurde bei 85°C geschmolzen, Phase II wurde zugefügt und die Temperatur auf 80°C gehalten. Phase III wurde kurz vor dem Einfüllen in die Gießform (welche mit Dimethicon 50 cts befeuchtet und auf 40 °C vorgeheizt war) zugegeben. Die Masse wurde in die Gießform gegeben und auf 40 °C abgekühlt. Die Gießformen wurde im Gefrierschrank auf nahe 0 °C abgekühlt.

**Beispiel 3.3 Styling Wachs**

| Phase | Komponente | INCI | Gew.-% |
|---|---|---|---|
| | Handelsname | | |
| I. | CUTINA® MD | Glyceryl Stearate | 14,50 |
| | COMPERLAN® 100 | Cocamide MEA | 2,50 |
| | CUTINA® HR | | |
| | Powder | Hydrogenated Castor Oil | 2,50 |
| | PLANTACARE® | | |
| | 1200 UP | Lauryl Glucoside | 5,00 |
| | LANETTE® O | Cetearyl Alcohol | 7,00 |
| | CUTINA® CP | Cetyl Palmitate | 7,00 |
| | EUMULGIN® O 20 | Cetoleth-20 | 5,00 |
| | Undecane/Tridecane | | 23,50 |
| | Vaseline | Petrolatum | 32,50 |
| | Wacker Siliconoil AK | | |
| | 350 | Dimethicone | 0,50 |

Die Herstellung erfolgt durch Erhitzen aller Komponenten auf 80 °C und Homogenisierung.

**Beispiel 3.4. Feuchtigkeitsspendende Körpermilch**

| Phase | Komponente | INCI | |
|---|---|---|---|
| | Handelsname | | Gew.-% |
| | | Cetearyl Isononanoate (and) Ceteareth- | |
| | | 20 (and) Cetearyl Alcohol (and)Glyceryl | |
| I. | EMULGADE® CM | Stearate (and) Glycerin (and) Ceteareth | |
| | | | 5.0 |
| | EUMULGIN® VL 75 | Lauryl Glucoside (and) Polyglyceryl-2 | |
| | | Dipolyhydroxystearate (and) Glycerin | 2.0 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| | Undecane/Tridecane | | 7.0 |
| II. | Wasser, deionisiert | | ad 100 |
| III. | Sepigel 305 (Seppic) | Polyacrylamide | 1.0 |
| IV. | HISPAGEL® 200 | Glycerin (and) Glyceryl Polyacrylate | 20.0 |
| | Konservierugsmittel, | | |
| V. | Parfum | | q.s. |
| | pH | 5.5 | |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert eingestellt.

**Beispiel 3.5 O/W Soft Creme**

| Phase | Komponente | INCI | |
|---|---|---|---|
| | Handelsname | | Gew.-% |
| | | Glyceryl Stearate (and) Ceteareth-20 | |
| | EMULGADE® SE- | (and) Ceteareth-12 (and) Stearyl Alcohol | |
| I. | PF | (and) Ceteareth-20 (and) Distearyl Ether | 6.0 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Undecane/Tridecane | | 8.0 |
| | Jojoba Oil | Simmondsia Chinensis (jojoba) Seed Oil | 2.0 |
| | | Tocopheryl Acetate | |
| | COPHEROL® 1250 | | 0.5 |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH | 5.5-6.5 | |

Diese Creme wurde hergestellt, indem Phase I auf 80°C erhitzt wurde, Phase II wurde ebenfalls auf 80°C erhitzt und zu Phase I unter Rühren hinzugefügt. Diese Mischung wurde unter Rühren abgekühlt und bei ca. 55°C mit einem geeigneten Dispersionsgerät (z.B. Ultra Turrax) homogenisiert. Danach wurde Phase III unter kontinuierlichem Rühren hinzugefiigt, Phase IV wurde zugegeben und der pH-Wert eingestellt.

**Beispiel 3.6 O/W Creme**

| Phase | Komponente | INCI | Gew.-% |
|---|---|---|---|
| | Handelsname | | |
| I. | MONOMULS® 90 O 18 | Glyceryl Oleate | 2,00 |
| | | | |
| | LAMEFORM® TGI | Polyglyceryl 3 Diisostearate | 4,00 |
| | CETIOL® A | Hexyl Laurate | 12,00 |
| | Undecane/Tridecane | | 9,00 |
| | SIPOL® C 16/18 OR | Cetearyl Alcohol | 1,00 |
| | Beeswax | Beeswax | 3,00 |
| | Zinc stearate | Zinc Stearate | 2,00 |
| | Zinc Oxide | CI 77947 (or) Zinc Oxide | 15,00 |
| | Magnesium Sulphate | Magnesium Sulphate | 1,00 |
| | Glycerin | Glycerin | 3,00 |
| | Konservierungsmittel | | q,s, |
| | Benzyl Alcohol | Benzyl Alcohol | 0,40 |
| | HYDAGEN® B | Bisabolol | 0,50 |
| | Irgasan DP300 | Triclosan | 0,05 |
| | Wasser | aqua | 100,00 |

Die ersten 8 Komponenten wurden bei 85°C geschmolzen. Magnesium Sulfate und Glycerin wurden im Wasser gelöst und diese Mischung wurde auf 85 °C erhitzt. Diese wässrige Phase wurde zur Ölphase gegeben und dispergiert. Unter kontinuierlichen Rühren wurde bis auf 40°C abgekühlt und dann wurden Benzyl Alkohol, Hydagen B und Irgansan DP300 gemischt und zu der Emulsion gegeben. Unter Weiterem Rühren wurde bis auf 30°C abgekühlt und homogenisiert.

**Beispiel 3.7. "Body Wash" Reinigungsemulsion**

| Phase | Komponente | INCI | |
|---|---|---|---|
| | Handelsname | | Gew.-% |
| I. | Texapon ALS-IS | Ammonium Lauryl | |
| | | Sulfate | 30,00 |
| | TEXAPON® NSO | Sodium Laureth | |
| | | Sulfate | 18,00 |
| | Undecane/Tridecane | | 18,00 |
| | Plantacare® 1200 | Lauryl Glucoside | |
| | | | 8,00 |
| II. | Jaguar HP 105 | Hydroxypopyl Guar | 2,00 |
| | | Methyldibromo | |
| | | Glutaronitrile and | |
| | Euxyl K400 | Phenoxyethanol | 0,10 |
| | Wasser | Aqua | 23,90 |
| | pH-value | 5,6 | |

**Tabelle 1: O/W-Sonnenschutzemulsionen**

| Die im Folgenden genannten Beispiele enthalten alle jeweils entweder Undecan (erhalten gemäß Beispiel 2) oder Tridecan (erhalten nach Beispiel 1). Alle Angaben sind in Gew.-%. | | | | | | | | | | | |
|---|---|---|---|---|---|---|---|---|---|---|---|
| | | | | | | | | | | | |
| **Komponente** | **1** | **2** | **3** | **4** | **5** | **6** | **7** | **8** | **9** | **10** | **11** |
| **L = Lotion, C = Creme,** | **L** | **C** | **S** | **L** | **C** | **L** | **L** | **C** | **L** | **C** | **L** |
| Eumulgin^{®} VL 75 | | | | | | 4 | 4 | 2 | | | |
| Eumulgin^{®} B2 | 2 | | | | | | | | | | |
| Tween^{®} 60 | | | | 1 | | | | | | | |
| Myrj^{®} 51 | | 3 | | 2 | | | | | | | |
| Cutina^{®} E 24 | 1 | | | 1 | | | | | | | |
| Hostaphat^{®} KL 340 N | | | | | | | | | 2 | | |
| Lanette^{®} E | | | 0.5 | | | | | 0.5 | | | |
| Amphisol^{®} K | | | 1 | | | 1 | | 0.5 | | 1 | |
| Natriumstearat | | | | | | | 1 | | | | 2 |
| Emulgade^{®} PL 68/50 | | | 1 | | 5 | | | | | 4 | |
| Tego^{®} Care 450 | | | | | | | | | | 3 | |
| Cutina^{®} MD | 2 | | | 6 | | | 4 | | | 6 | |
| Lanette^{®} 14 | 1 | | | 1 | | | | 2 | | | 4 |
| Lanette^{®} O | 1 | 6 | | | 5 | 2 | | 2 | | | |
| Antaron V 216 | | | 1 | | 2 | 2 | | | | 1 | |
| Emery^{®} 1780 | | | | | 0.5 | 0.5 | | | | | |
| Lanolin, wasserfrei USP | | | | | | | 5 | | | | |
| Undecan oder Tridecan | 2 | 2 | 4 | 1 | 2 | 2 | 2 | 1 | 2 | 2 | 1 |
| Myritol^{®} PC | | | | | 5 | | | | | | |
| Myritol^{®} 331 | 5 | | 8 | | | 6 | | 10 | | 2 | |
| Finsolv^{®} TN | | | 1 | | | | | 1 | 8 | | |
| Cetiol^{®} CC | | 2 | 5 | | | 4 | 4 | 2 | | 2 | |
| Cetiol^{®} OE | | | 3 | | | | | | 2 | 3 | |
| Dow Corning DC^{®} 244 | 4 | | 1 | | 5 | | | 2 | | | 2 |
| Dow Corning DC^{®} 2502 | | 1 | | | 2 | | | | | | |
| Squatol^{®} S | | | | | | | 4 | | | | |
| Silikonöl Wacker AK^{®} 350 | | 2 | | | | | | | | | |
| Cetiol^{®} 868 | | | | | 2 | | 4 | | | | 7 |
| Cetiol^{®} J 600 | | | | | 3 | 2 | | | | 5 | |
| Mineralöl | | | | 9 | | | | | | | |
| Cetiol^{®} B | | | 1 | | | | | | | 2 | |
| Eutanol^{®} G | | | | | | | | | | | |
| Eutanol^{®} G 16 | | | | | | | | | | | |
| Cetiol^{®} PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl^{®} LS | | | | | | | | | | | |
| Neo Heliopan^{®} Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | | | 0.7 | | | | | 1 | 1 | | |
| Keltrol^{®} T | | | 0.2 | | | | | 0.5 | 0.5 | | |
| Carbopol^{®} 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | ad 100 | | | | | | | | | | |

**Tabelle 2: O/W-Sonnenschutzemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin^{®} VL 75 | 4 | 3 | 4.5 | | 3 | | | | 4 | | |
| Eumulgin^{®} B2 | | | | | | | | | | 1 | |
| Tween^{®} 60 | | | | | | | | | | 1 | |
| Myrj^{®} 51 | | | | | | | | | | | |
| Cutina^{®} E 24 | | | | 2 | | | | | | | |
| Hostaphat^{®} KL 340 N | | | | | | | | | | 0.5 | |
| Lanette^{®} E | 0.5 | | 0.5 | 0.5 | | | 0.1 | | 0.5 | | |
| Amphisol^{®} K | 0.5 | | | | | 1 | 1 | 1 | | | |
| Natriumstearat | | | | | 1 | | | | | | |
| Emulgade^{®} PL 68/50 | | 6 | | | | 4.5 | 1 | 5 | | | |
| Tego^{®} Care 450 | 1 | | | | | | | | 4 | | |
| Cutina^{®} MD | 1 | | | 8 | 6 | 1 | | | | 4 | 1 |
| Lanette^{®} 14 | | 2 | | | | | | 2 | | 1 | |
| Lanette^{@} O | | | | 2 | | | | | 1 | 1 | |
| Antaron V 220 | 1 | | | 2 | | | 0.5 | | | 2 | 0.5 |
| Undecan oder Tridecan | 4 | 2 | 4 | 6 | 10 | 4 | 2 | 8 | 2 | 1 | 3 |
| Myritol^{®} PC | | | | | | | | | 5 | | |
| Myritol^{®} 331 | 12 | | 12 | | | 8 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Corning DC^{®} 244 | | 2 | | | 1 | | | | | | |
| Dow Corning DC^{®} 2502 | | 1 | | | 1 | | | | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 10 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Neo Heliopan AP (Na-Salz) | | 2 | | 2 | | | 2 | | | | 1 |
| Eusolex^{®} OCR | 6 | | 9 | | 5 | 7 | 9 | | 4 | | 7 |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} T 150 | 1 | | | | | | | | 1.3 | 1 | 1 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Pemulen^{®} TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 3: W/O-Sonnenschutzemulsionen**

| **Komponente** | **23** | **24** | **25** | **26** | **27** | **28** | **29** | **30** | **31** | **32** | **33** |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls^{®} PGPH | 4 | 2 | 1 | 3 | 3 | 1 | 1 | 2 | 2 | 4 | 1 |
| Monomuls^{®} 90-018 | | | 2 | | | | | | | | |
| Lameform^{®} TGI | 2 | | 4 | | 3 | | | | | 1 | 3 |
| Abil^{®} EM 90 | | | | | | | 4 | | | | |
| Glucate^{®} DO | | | | | | | | | | | 3 |
| Isolan^{®} PDI | | | | | | 4 | | 2 | | | |
| Arlacel^{®} 83 | | | | 2 | | | | | | | |
| Elfacos^{®} ST9 | | | | | | | | | 2 | | |
| Elfacos^{®} ST37 | | | | | | | | | | | |
| Arlacel^{®} P 135 | | 2 | | | | | | | | | |
| Dehymuls^{®} HRE 7 | | | | | | | | | | | |
| Zinkstearat | 1 | | | 1 | 1 | | | 1 | | 1 | |
| Microkristallines Wachs | | | 5 | | | 2 | | | | | 5 |
| Bienenwachs | 1 | | | 1 | | | | 5 | | 7 | |
| Tego^{®} Care CG | | | | | 1 | | | | | | .5 |
| Prisorine^{®} 3505 | 1 | | 1 | 1 | | 1 | 1 | | | | 1 |
| Emery^{®} 1780 | | | 5 | | | | | | | 4 | |
| Wollwachsalkohol, wasserfrei, | | | | | | | | | | | 1 |
| Antaron V 216 | 2 | | | | | | | | | | |
| Undecan oder Tridecan | 3 | 4 | 2 | 1 | 10 | 2 | 2 | 6 | 3 | 12 | 1 |
| Myritol^{®} PC | | | | | 3 | | | 4 | | | |
| Myritol^{®} 331 | 10 | | | | 3 | 6 | | | | | 8 |
| Finsolv^{®} TN | | | | 5 | | | 5 | | | | |
| Cetiol^{®} CC | 12 | 22 | | | | 2 | | | 2 | | 5 |
| Cetiol^{®} OE | | | | | 4 | | 5 | | 4 | 2 | |
| Dow Corning DC^{®} 244 | | | | | | | 2 | | | | |
| Dow Corning DC^{®} 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol^{®} 868 | | | | | | | | | | 2 | |
| Eutanol^{®} G 16 | | 3 | | | | | | | | | |
| Eutanol^{®} G 16S | | | | | | | | | | | |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G | | | 3 | | | | | 8 | | | |
| Cetiol^{®} PGL | | 11 | | | | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 4: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion; C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 2 | 4 | 0.5 | 1 | 4 |
| Monomuls^{®} 90-018 | | 1 | | | | | | | | | |
| Lameform^{®} TGI | | | | | 4 | | | 1 | | 3 | 1 |
| Abil^{®} EM 90 | | | | 1 | | | | | | 2 | |
| Glucate^{®} DO | | | | 3 | | | | | 2 | | |
| Isolan^{®} PDI | | 3 | | | | | 4 | | | | |
| Arlacel^{®} 83 | | | | | | 3 | | | | | |
| Elfacos^{®} ST9 | | | | | | | | | | | 2 |
| Elfacos^{®} ST37 | 2 | | | | | | | | | | |
| Arlacel^{®} P 135 | | | | | | 3 | | | | | |
| Dehymuls^{®} HRE 7 | | | | | | | | | 4 | | |
| Zinkstearat | | 2 | 2 | 1 | 1 | | | 1 | 1 | | |
| Mikrokristallines Wachs | | | | | 4 | | 1 | | | 4 | |
| Bienenwachs | | 4 | | 2 | | | 1 | 1 2 | 2 | | 1 |
| Tego^{®} Care CG | | | | | | | | | | | |
| Isostearinsäure | 1 | 1 | | | | | 1 | 1 | | 1 | 1 |
| Emery^{®} 1780 | | 7 | 3 | | | | | | | | |
| Wollwachsalkohol, wasserfrei, | | | | | | | | | | | |
| Antaron V 220 | | 0.5 | 2 | 1 | 1 | 1 | | | | | |
| Undecan oder Tridecan | 2 | 4 | 3 | 3 | 2 | 2 | 1 | 3 | 3 | 1 | 4 |
| Myritol^{®} PC | | | | | | | | | | | |
| Myritol^{®} 331 | 4 | 2 | 3 | | 5 | | | 8 | 5 | 4 | |
| Finsolv^{®} TN | | 5 | 5 | | | 7 | | | | | |
| Cetiol^{®} CC | 3 | 1 | | | | | 3 | 16 | | | 12 |
| Cetiol^{®} OE | | 3 | | 2 | | | 3 | | | | |
| Dow Corning DC^{®} 244 | | 4 | | 2 | | | | | | | |
| Dow Corning DC^{®} 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol^{®} 868 | | | | | | | | | | | |
| Eutanol^{®} G 16 | | | | | | | | | | | 3 |
| Eutanol^{®} G 16S | | | | | | | | | | | 7 |
| Cetiol^{®} J 600 | | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G | | | | 2 | | | | | 5 | | |
| Cetiol^{®} PGL | | | | | | | | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan^{®} Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan^{®} MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote^{®} HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen**

| **Komponente** | **45** | **46** | **47** | **48** | **49** | **50** | **51** | **52** | **53** | **54** | **55** |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls^{®} PGPH | 1 | 3 | 1 | 2 | 3 | 1 | 1 | 2 | 1 | 1 | 1 |
| Monomuls^{®} 90-018 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| Undecan oder Tridecan | 3 | 4 | 2 | 12 | 10 | 2 | 2 | 6 | 3 | 12 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N, N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 6: W/O-Pflegeemulsionen**

| **Komponente** | **56** | **57** | **58** | **59** | **60** | **61** | **62** | **63** | **64** | **65** | **66** |
|---|---|---|---|---|---|---|---|---|---|---|---|
| **L = Lotion. C = Creme** | **L** | **C** | **L** | **L** | **C** | **L** | **L** | **L** | **L** | **C** | **C** |
| Dehymuls^{®} PGPH | 3 | 1 | 5 | 1 | 1 | 3 | 3 | 4 | 1 | 1 | 1 |
| Monomuls^{®} 90-018 | | 1 | | 1 | | | | | | | |
| Lameform^{®} TGI | | | | | 4 | | | 1 | | 3 | |
| Abil^{®} EM 90 | | | | 3 | | | | | | 2 | |
| Isolan^{®} PDI | | 3 | | | | | | | | | 4 |
| Glucate^{®} DO | 1 | | | | | | | | | | |
| Arlacel^{®} 83 | | | | | | 3 | | | | | |
| Dehymuls^{®} FCE | | | | | 4 | | 1 | | | | |
| Dehymuls^{®} HRE 7 | | | | | | | | | 7 | | |
| Zinkstearat | | 2 | 2 | 1 | 1 | 1 | | 1 | 1 | | 1 |
| Mikrokristallines Wachs | | | | | 4 | | 1 | | | 4 | |
| Bienenwachs | | 4 | | 2 | | 2 | 1 | 1 | 2 | | 5 |
| Tego^{®} Care CG | | | | | | | | | | | |
| Prisorine^{®} 3505 | 1 | 1 | | | | | 1 | 1 | | 1 | 1 |
| Dry Flo^{®} Plus | 1 | | | | | | | | | | |
| SFE^{®} 839 | | 5 | | | | 4 | | | | | |
| Emery^{®} 1780 | | | | | | | | | | | |
| Lanolin anhydrous USP | | 7 | 3 | | | | | | | | |
| Undecan oder Tridecan | 3 | 4 | 4 | 8 | 10 | 2 | 8 | 6 | 3 | 12 | 7 |
| Cegesoft^{®} C 17 | | 2 | | | | | | | | | |
| Myritol^{®} PC | | | | 8 | | | | | | | |
| Myritol^{®} 331 | 4 | | 3 | | 5 | 3 | | | 5 | 4 | |
| Finsolv^{®} TN | | | 5 | | | 7 | | | | | |
| Cetiol^{®} A | | | | | | | | 6 | | | |
| Cetiol^{®} CC | 3 | | | 6 | | 3 | 3 | | | 8 | |
| Cetiol^{®} SN | | | | | 5 | | | | | | |
| Cetiol^{®} OE | | 3 | | 2 | | | 3 | | | | 8 |
| Dow Corning^{®} DC 244 | | 4 | | 2 | | 2 | | | | | |
| Dow Corning^{®} DC 2502 | | | | 1 | | | | | | | |
| Prisorine^{®} 3758 | | | | | | 1 | | | | | |
| Silikonöl Wacker AK^{®} 350 | | | | 1 | | 1 | | 4 | | | |
| Cetiol^{®} 868 | | | | | | | | | | | 10 |
| Cetiol^{®} J 600 | 4 | | | 3 | | | | | | | |
| Ceraphyl^{®} 45 | | | | 1 | | | | | 5 | 4 | |
| Mineralöl | | | | | | | 9 | | | | |
| Cetiol^{®} B | | | | | 3 | 3 | | 2 | 2 | | |
| Eutanol^{®} G 16 | 1 | | | | | | | | | | |
| Eutanol^{®} G | | | | 2 | | | | | 5 | | |
| Cetiol^{®} PGL | | | 10 | | | | | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen**

| **Komponente** | **67** | **68** | **69** | **70** | **71** | **72** | **73** | **74** | **75** | **76** | **77** |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Eumulgin^{®} VL 75 | | | | | | 4 | | | | | |
| Dehymuls^{®} PGPH | | 2 | | | | | | | | | |
| Generol^{®} R | | | 1 | | | | | | | | |
| Eumulgin^{®} B2 | | | 0.8 | | | | | | | | |
| Tween^{®} 60 | | | | 1 | | | | | | | |
| Cutina^{®} E 24 | | | 0.6 | 2 | | | | | | | |
| Hostaphat^{®} KL 340 N | | | | | | | | | 2 | | |
| Lanette^{®} E | | | | | | | | 1 | | | |
| Amphisol^{®} K | | 0.5 | | | | 1 | | | | 1 | 0.5 |
| Natriumstearat | | | | | 0.5 | | | | | | |
| Emulgade^{®} PL 68/50 | | 2.5 | | | | | | | | 4 | |
| Tego^{®} Care CG | | | | | | | | | | | 2 |
| Tego^{®} Care 450 | | | | | | | | 5 | | | |
| Cutina^{®} MD | | 1 | | 6 | 5 | | 4 | | | 6 | |
| Lanette^{®} 14 | | | | 1 | | | | 2 | | | 4 |
| Lanette^{@} O | 4.5 | | 4 | | 1 | 2 | | | | | 2 |
| Novata^{®} AB | | 1 | | | | | | | | | 1 |
| Emery^{®} 1780 | | | | | 0.5 | 0.5 | | | | | |
| Lanolin, wasserfrei, USP | | | | | | | 5 | | | | |
| Cetiol^{®} SB 45 | | | 1.5 | | | | 2 | | | | |
| Undecan oder Tridecan | 3 | 4 | 2 | 1 | 10 | 2 | 2 | 6 | 3 | 12 | 1 |
| Cegesoft^{®} C 17 | | | | | | | | | | | |
| Myritol^{®} PC | | | | | 5 | | | | | | |
| Myritol^{®} 331 | 2 | 5 | 5 | | | 6 | | 12 | | | |
| Finsolv^{®} TN | | | 2 | | | 2 | | | 8 | | |
| Cetiol^{®} CC | 4 | 6 | | | | 4 | 4 | | | | 5 |
| Cetiol^{®} OE | | | | | | | | | 4 | 3 | |
| Dow Corning DC^{®} 245 | | | 2 | | 5 | 1 | | | | | |
| Dow Corning DC^{®} 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineralöl | | | | 9 | | | | | | | |
| Cetiol^{®} SN | | | 5 | | | | | | | | |
| Cetiol^{®} B | | | | | | | | | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N, N-Diethyl-m-toluamid | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} ultra | | | | | | | | | 1 | | |
| Keltrol^{®} T | | | 0.4 | | | | | | 0.5 | | |
| Pemulen^{®} TR 2 | 0.3 | | | | | | | 0.3 | | | |
| Carbopol^{®} Ultrez 10 | | 0.3 | 0.3 | 0.2 | 0.2 | 0.2 | | | 0.1 | 0.3 | 0.2 |
| Ethanol | | | | | | | | | | 10 | |
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., pH 6,5 - 7,5 | | | | | | | | | | |

**Tabelle 8: O/W-Pflegeemulsionen**

| **Komponente** | **78** | **79** | **80** | **81** | **82** | **83** | **84** | **85** | **86** | **87** | **88** |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin^{®} VL 75 | 4 | 3 | | | | | 1 | | | | 2 |
| Generol^{®} R | | | | | | 2 | | | | | |
| Eumulgin^{®} B2 | | | | | | 2 | | | | 1 | |
| Tween^{®} 60 | | | | | | | | | | 1 | |
| Cutina^{®} E 24 | | | | 2 | | | | | | | |
| Hostaphat^{®} KL 340 N | | | | | | | | | | | |
| Lanette^{®} E | 0.5 | | | | | | | | | | 1 |
| Amphisol^{®} K | 0.5 | 1 | | | | | | 1 | 1 | | |
| Natriumstearat | | | | | 1 | | | | | | |
| Emulgade^{®} PL 68/50 | | 6 | | | | | | 5 | | | 4 |
| Tego^{®} Care CG | | | | | | | | | | | |
| Tego^{®} Care 450 | | | | | | | | | 4 | | |
| Cutina^{®} MD | 3 | | 3 | 8 | 6 | 8 | | | | 4 | |
| Lanette^{®} 14 | | 2 | | | | | | 2 | | 1 | |
| Lanette^{@} O | 2 | | | 2 | | 3 | 1 | | 1 | 1 | 6 |
| Novata^{®} AB | | | | | | | | | | | |
| Emery^{®} 1780 | | | | | | | | | | | |
| Lanolin, wasserfrei, USP | | | | | | 4 | | | | | |
| Cetiol^{®} SB 45 | | | | | | | 2 | | | | |
| Undecan oder Tridecan | 3 | 4 | 2 | 1 | 10 | 2 | 2 | 6 | 3 | 12 | 1 |
| Cegesoft^{®} C 17 | 4 | | | | | | | | | | |
| Myritol^{®} PC | 6 | | | | | 5 | | | 5 | | |
| Myritol^{®} 331 | 5 | | 5 | | | | 7 | | | 10 | 3 |
| Finsolv^{®} TN | | 5 | | | 5 | | | 3 | 3 | | 1 |
| Cetiol^{®} CC | | | | | | | | | | | 2 |
| Cetiol^{®} OE | | | | | 2 | | 2 | | 5 | | |
| Dow Corning DC^{®} 245 | | 2 | | | 1 | | | | | 8 | 2 |
| Dow Corning DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | | | | | | | | |
| Ceraphyl^{®} 45 | | | | | | | 3 | | | | |
| Cetiol^{®} SN | | | | | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Carbopol^{®} ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Tabelle 9: Sprayformulierungen**

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* = Sonnenschutzspray** | **S** | **S** | **S** | **S** | **S** | **S*** | **S*** | **S*** | **5*** | **S*** | **S*** |
| Emulgade^{®} SE-PF | 8,9 | | 7,5 | 7.5 | 4,3 | 9.8 | 8.2 | 9.9 | | | |
| Eumulgin^{®} B2 | 3,1 | | 3 | | | | | 4.2 | | | |
| Eumulgin^{®} B3 | | | | | | 4.2 | 3.3 | | | | |
| Eumulgin^{®} HRE 40 | | | | | 4,7 | | | | | | |
| Cutina^{®} E 24 | | 5,9 | | 4 | | | | | | | |
| Amphisol^{®} K | | | | | | | | | 1 | 1 | 1 |
| Eumulgin^{®} VL 75 | | | | | | | | | | | 2 |
| Emulgade^{®} PL 68/50 | | 0.5 | | | | | | | 2.5 | 1 | |
| Cutina^{®} MD | | 3,1 | | | | | | | | | |
| Antaron V 220 | | | | | | 1 | 1 | 1 | | 1 | 1 |
| Undecan oder Tridecan | 11 | 5 | 7 | 7 | 7 | 5 | 4 | 5 | 5 | 4 | 6 |
| Myritol^{®} PC | | | | | | | | | | | |
| Myritol^{®} 331 | | | 3 | 4 | 3 | 3 | 3 | 3 | | | |
| Finsolv^{®} TN | | 4 | | | | | | 8 | | | |
| Cetiol^{®} CC | 6 | | | 5 | 5 | 2 | 2 | 4 | | | |
| Cetiol^{®} OE | | 5 | 5 | | | 2 | | | | | |
| Dow Corning DC^{®} 244 | | 4 | 4 | 5 | | | | | | | |
| Cetiol^{®} 868 | 3 | | | | | | | | | | |
| Cetiol^{®} J 600 | | | | 2 | 2 | | | | | | |
| Mineralöl | | | 2 | | | | | | | | |
| Cetiol^{®} B | | | | | | | 2 | | | | |
| Eutanol^{®} G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan^{®} MBC | | | | | | 2 | 2 | 2 | | 1 | 1 |
| Neo Heliopan^{®} OS | | | | | | 5 | | | | | |
| Neo Heliopan^{®} AV | | | | | | 6 | 6 | 2 | | 7.5 | 2 |
| Uvinul^{®} T 150 | | | | | | 1 | 1 | 1 | | 1 | |
| Parsol^{®} 1789 | | | | | | 1 | | 1 | | 1 | |
| Z-Cote^{®} HP 1 | | | | | | | | | | 2 | 2 |
| Eusolex^{®} T 2000 | | | | | | | | | | 2 | 2 |
| Veegum^{®} Ultra | | | | | | | | | | | 1.5 |
| Laponite^{®}XLG | | | | | | | | | | 1.5 | |
| Keltrol^{®} T | | | | | | | | | | | 0.5 |
| Pemulen^{®} TR 2 | | | | | | | | | 0.2 | | |
| Insect Repellent^{®} 3535 | 1 | | | | | | | | | | |
| N, N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.s./ q.s | | | | | | | | | | |

**ANHANG**

| | |
|---|---|
| 1) Abil^{®} EM 90 | |
| INCI: Cetyl Dimethicone Copolyol | 12) Cegesoft^{®} C 17 |
| Hersteller: Tego Cosmetics (Goldschmidt) | INCI: Myristyl Lactate |
| | Hersteller: Cognis Deutschland GmbH, Grünau |
| 2) Amphisol^{®} K | |
| INCI: Potassium Cetyl Phosphate | 13) Ceraphyl^{®} 45 |
| Hersteller: Hoffmann La Roche | INCI: Diethylhexyl Malate |
| | Hersteller: International Specialty Products |
| 3) Antaron^{®} V 220 | |
| INCI: PVP/Eicosene Copolymer | 14) Cetiol^{®} 868 |
| Hersteller: GAF General Aniline Firm Corp. | INCI: Ethylhexyl Stearate |
| (IPS-Global) | Hersteller: Cognis Deutschland GmbH |
| | |
| 4) Antaron^{®} V 216 | 15) Cetiol^{®} A |
| INCI: PVP/Hexadecene Copolymer | INCI: Hexyl Laurate |
| Hersteller: GAF General Aniline Firm Corp. | Hersteller: Cognis Deutschland GmbH |
| (IPS-Global) | |
| | 16) Cetiol^{®} B |
| 5) Arlacel^{®} 83 | INCI: Butyl Adipate |
| INCI: Sorbitan Sesquioleate | Hersteller: Cognis Deutschland GmbH (Henkel) |
| Hersteller: Uniqema (ICI Surfacants) | |
| | 17) Cetiol^{®} J 600 |
| 6) Arlacel^{®} P 135 | INCI: Oleyl Erucate |
| INCI: PEG-30 Dipolyhydroxystearate | Hersteller: Cognis Deutschland GmbH |
| Hersteller: Uniqema (ICI Surfacants) | |
| | 18) Cetiol^{®} OE |
| 7) Bentone^{®} 38 | INCI: Dicaprylyl Ether |
| INCI: Quaternium-18 Hectorite | Hersteller: Cognis Deutschland GmbH |
| Hersteller: Rheox (Elementis Specialties) | |
| | 19) Cetiol^{®} PGL |
| 8) Carbopol^{®} 980 | INCI: Hexyldecanol, Hexyldecyl Laurate |
| INCI: Carbomer | Hersteller: Cognis Deutschland GmbH |
| Hersteller: Goodrich | |
| | 20) Cetiol^{®} CC |
| 9) Carbopol^{®} 2984 | INCI: Dicaprylyl Carbonate |
| INCI: Carbomer | Hersteller: Cognis Deutschland GmbH |
| Hersteller: Goodrich | |
| | 21) Cetiol^{®} SB 45 |
| 10) Carbopol^{®} ETD 2001 | INCI: Shea Butter Butyrospermum Parkii |
| INCI: Carbomer | (Linne) |
| Hersteller: BF Goodrich | Hersteller: Cognis Deutschland GmbH |
| | |
| 11) Carbopol^{®} Ultrez 10 | 22) Cetiol^{®} SN |
| INCI: Carbomer | INCI: Cetearyl Isononanoate |
| Hersteller: Goodrich | Hersteller: Cognis Deutschland GmbH (Henkel) |
| 23) Cutina^{®} E 24 | 35) Emulgade^{®} PL 68/50 |
| INCI: PEG-20 Glyceryl Stearate | INCI: Cetearyl Glucoside, Ceteayl Alcohol |
| Hersteller: Cognis Deutschland GmbH | Hersteller: Cognis Deutschland GmbH |
| 24) Cutina^{®} MD | |
| INCI: Glyceryl Stearate | |
| Hersteller: Cognis Deutschland GmbH | 36) Emulgade^{®} SE-PF |
| | INCI: Glyceryl Stearate, Ceteareth-20, |
| 25) Dehymuls^{®} FCE | Ceteareth-12, Cetearyl Alcohol, Cetyl Palmitate |
| INCI: Dicocoyl Pentaerythrityl Distearyl | Hersteller: Cognis Deutschland GmbH |
| Citrate | |
| Hersteller: Cognis Deutschland GmbH | 37) Eumulgin^{®} B 2 |
| | INCI: Ceteareth- 20 |
| 26) Dehymuls^{®} HRE 7 | Hersteller: Cognis Deutschland GmbH |
| INCI: PEG-7 Hydrogenated Castor Oil | |
| Hersteller: Cognis Deutschland GmbH | 38) Eumulgin^{®} VL 75 |
| | INCI: Lauryl Glucoside (and) Polyglyceryl-2 |
| 27) Dehymuls^{®} PGPH | Dipolyhydroxystearate (and) Glycerin |
| INCI: Polyglyceryl-2 Dipolyhydroxystearate | Hersteller: Cognis Deutschland GmbH |
| Hersteller: Cognis Deutschland GmbH | |
| | 39) Eusolex^{®} OCR |
| 28) Dow Corning^{®} 244 Fluid | INCI: Octocrylene |
| INCI: Cyclomethicone | Hersteller: Merck |
| Hersteller: Dow Corning | |
| | 40) Eusolex^{®} T 2000 |
| 29) Dow Corning^{®} 245 Fluid | INCI: Titanium Dioxide, Alumina, |
| INCI: Cyclopentasiloxane Cyclomethicone | Simethicone |
| Hersteller: Dow Corning | Hersteller: Rona (Merck) |
| | |
| 30) Dow Corning^{®} 2502 | 41) Eutanol^{®} G |
| INCI: Cetyl Dimethicone | INCI: Octyldodecanol |
| Hersteller: Dow Corning | Hersteller: Cognis Deutschland GmbH |
| | |
| 31) Dry^{®}Flo Plus | 42) Eutanol^{®}G 16 |
| INCI: Aluminium Starch Octenylsuccinate | INCI: Hexyldecanol |
| Hersteller: National Starch | Hersteller: Cognis Deutschland GmbH |
| 32) Elfacos^{®}ST 37 | 43) Eutanol^{®}G 16 S |
| INCI: PEG-22 Dodecyl Glycol Copolymer | INCI: Hexyldecyl Stearate |
| Hersteller: Akzo-Nobel | Hersteller: Cognis Deutschland GmbH |
| | |
| 33) Elfacos^{®}ST 9 | 44) Finsolv^{®} TN |
| INCI: PEG-45 Dodecyl Glycol Copolymer | INCI: C 12/15 Alkyl Benzoate |
| Hersteller: Akzo-Nobel | Hersteller: Findex (Nordmann/Rassmann) |
| | |
| 34) Emery^{®} 1780 | 45) Generol^{®} R |
| INCI: Lanolin Alcohol | INCI: Brassica Campestris (Rapseed) Sterols |
| Hersteller: Cognis Corporation (Emery) | Hersteller: Cognis Deutschland GmbH |
| 46) Glucate^{®} DO | 57) Neo Heliopan^{®} 303 |
| INCI: Methyl Glucose Dioleate | INCI: Octocrylene |
| Hersteller: NRC Nordmann/Rassmann | Hersteller: Haarmann & Reimer |
| | |
| 47) Hostaphat^{®} KL 340 N | 58) Neo Heliopan^{®} AP |
| INCI: Trilaureth -4 Phosphate | INCI: Disodium Phenyl Dibenzimidazole |
| Hersteller: Clariant | Tetrasulfonate |
| 48) Isolan^{®} PDI | Hersteller: Haarmann & Reimer |
| INCI: Diisostearoyl Polyglyceryl-3 | |
| Diisostearate | 59) Neo Heliopan^{®} AV |
| Hersteller: Goldschmidt AG | INCI: Ethylhexyl Methoxycinnamate |
| | Hersteller: Haarmann & Reimer |
| 49) Keltrol^{®} T | |
| INCI: Xanthan Gum | 60) Neo Heliopan^{®} BB |
| Hersteller: CP Kelco | INCI: Benzophenone-3 |
| | Hersteller: Haarmann & Reimer |
| 50) Lameform^{®} TGI | |
| INCI: Polyglyceryl-3 Diisostearate | 61) Neo Heliopan^{®} E 1000 |
| Hersteller: Cognis Deutschland GmbH | INCI: Isoamyl-p-Methoxycinnamate |
| | Hersteller: Haarmann & Reimer |
| 50) Lanette^{®} 14 | |
| INCI: Myristyl Alcohol | 62) Neo Heliopan^{®} Hydro (Na-Salz) |
| Hersteller: Cognis Deutschland GmbH | INCI: Phenylbenzimidazole Sulfonic Acid |
| | Hersteller: Haarmann & Reimer |
| 51) Lanette^{®} E | |
| INCI: Sodium Cetearyl Sulfate | 63) Neo Heliopan^{®} MBC |
| Hersteller: Cognis Deutschland GmbH | INCI: 4-Methylbenzylidene Camphor |
| | Hersteller: Haarmann & Reimer |
| 52) Lanette^{®} O | |
| INCI: Cetearyl Alcohol | 64) Neo Heliopan^{®} OS |
| Hersteller: Cognis Deutschland GmbH | INCI: Ethylhexyl Salicylate |
| | Hersteller: Haarmann & Reimer |
| 53) Monomuls^{®} 90-0-18 | |
| INCI: GlycerylOleate | 65) Novata^{®} AB |
| Hersteller: Cognis Deutschland GmbH | INCI: Cocoglycerides |
| | Hersteller: Cognis Deutschland GmbH |
| 54) Myrj^{®} 51 | |
| INCI: PEG-30-Sterate | 66) Parsol^{®} 1789 |
| Hersteller: Uniqema | INCI: Butyl Methoxydibenzoylmethane |
| | Hersteller: Hoffmann-La Roche (Givaudan) |
| 55) Myritol^{®} 331 | |
| INCI: Cocoglycerides | 67) Pemulen^{®} TR-2 |
| Hersteller: Cognis Deutschland GmbH | INCI: Acrylates / C10-30 Alkylacrylate |
| | Crosspolymer |
| 56) Myritol^{®} PC | Hersteller: Goodrich |
| INCI: Propylene Glycol Dicaprylate/Dicaprate | |
| Hersteller: Cognis Deutschland GmbH | 68) Photonyl^{®} LS |
| INCI: Arginine, Disodium Adenosine | |
| Triphosphate, Mannitol, Pyridoxine HCL, | 80) Veegum^{®} Ultra |
| Phenylalanine, Tyrosine | INCI: Magnesium Aluminium Silicate |
| Hersteller: Laboratoires Serobiologiques | Hersteller: Vanderbilt |
| (Cognis) | |
| | 81) Z-Cote^{®} HP 1 |
| 69) Prisorine^{®} ISAC 3505 | INCI: Zinc Oxide, Dimethicone |
| INCI: Isostearic Acid | Hersteller: BASF |
| Hersteller: Uniqema | |
| 70) Prisorine^{®} 3758 | |
| INCI: Hydrogenated Polyisobutene | |
| Hersteller: Uniqema | |
| 71) Ravecarb^{®} 106 | |
| Polycarbonatdiol | |
| Hersteller: Enichem | |
| 73) SFE^{®} 839 | |
| INCI: Cyclopentasiloxane and | |
| Dimethicone/Vinyl Dimethicone Crosspolymer | |
| Hersteller: GE Silicones | |
| 74) Silikonöl Wacker AK^{®} 350 | |
| INCI: Dimethicone | |
| Hersteller: Wacker | |
| 75) Squatol^{®} S | |
| INCI: Hydrogenated Polyisobutene | |
| Hersteller: LCW (7-9 rue de l'Industrie 95310 | |
| St-Ouen l'Aumone France) | |
| 76) Tego^{®} Care 450 | |
| INCI: Polyglyceryl-3 Methylglucose | |
| Distearate | |
| Hersteller: Tego Cosmetics (Goldschmidt) | |
| 77) Tego^{®} Care CG 90 | |
| INCI: Cetearyl Glucoside | |
| Hersteller: Goldschmidt | |
| 78) Tween^{®} 60 | |
| INCI: Polysorbate 60 | |
| Hersteller: Uniqema (ICI Surfactants) | |
| 79) Uvinul^{®} T 150 | |
| INCI: Octyl Triazone | |
| Hersteller: BASF | |

## Patentansprüche

1. Verfahren zur Herstellung von linearen gesättigten Alkanen aus primären Alkoholen, deren C-Kette ein Kohlenstoffatom mehr enthält als das Alkan durch reduktive Dehydroxymethylierung der primären Alkohole in Gegenwart von Wasserstoff und einem Nickel-haltigen Katalysator bei Temperaturen von 100 bis 300°C und Drücken von 2 bis 300 bar, wobei während der Reaktion entstehendes Wasser entfernt wird, **dadurch gekennzeichnet, dass** als primäre Alkohole Fettalkohole mit 8 bis 24 Kohlenstoffatomen ausgewählt sind und der Katalysator in Mengen von 0,1 bis 3 Gew.-% zugesetzt wird.

2. Verfahren nach Anspruch 1, **dadurch gekennzeichnet, dass** die Reaktion bei Temperaturen von 180 bis 300°C, vorzugsweise 200 bis 280°C und insbesondere von 220 bis 260°C durchgeführt wird.

3. Verfahren nach den Ansprüchen 1 bis 2, **dadurch gekennzeichnet, dass** das Verfahren bei Drücken von 2 bis 250 bar, insbesondere von 5 bis 100 bar, vorzugsweise 5 bis 80 bar und insbesondere von 10 bis 50 bar durchgeführt wird.

4. Verfahren nach den Ansprüchen 1 bis 3, **dadurch gekennzeichnet, dass** der Katalysator in Mengen von 0,2 bis 2 Gew.-% und insbesondere in Mengen von 0,5 bis 1,0 Ges.-%, jeweils bezogen auf die Menge an eingesetztem Fettalkohol, eingesetzt wird.

5. Verfahren nach den Ansprüchen 1 bis 4, **dadurch gekennzeichnet, dass** als primäre Alkohole solche der allgemeinen Formel R-OH ausgewählt sind, in der R für einen gesättigten, linearen Alkylrest mit 8 bis 18, vorzugsweise 10 bis 16 und insbesondere 12 bis 16 Kohlenstoffatomen steht.

6. Vorfahren nach einem der Ansprüche 1 bis 5, **dadurch gekennzeichnet, dass** die primären Alkohole eine geradzahlige Kohlenstoffanzahl enthalten.

7. Verfahren nach einem der Ansprüche 1 bis 6, **dadurch gekennzeichnet, dass** Mischungen der primären Alkohole eingesetzt werden.

8. Verfahren nach einem der Ansprüche 1 bis 7, **dadurch gekennzeichnet, dass** im Anschluss an die Reaktion das erhaltene Produkt gereinigt wird.

## Claims

1. Process for the preparation of linear saturated alkanes from primary alcohols whose C chain contains one carbon atom more than the alkane by reductive dehydroxymethylation of the primary alcohols in the presence of hydrogen and a nickel-containing catalyst at temperatures of 100 to 300°C and pressures of 2 to 300 bar, with water formed during the reaction being removed, **characterized in that** fatty alcohols having 8 to 24 carbon atoms are selected as primary alcohols and the catalyst is added in amounts of 0.1 to 3% by weight.

2. Process according to Claim 1, **characterized in that** the reaction is carried out at temperatures from 180 to 300°C, preferably 200 to 280°C and in particular from 220 to 260°C.

3. Process according to Claims 1 to 2, **characterized in that** the process is carried out at pressures from 2 to 250 bar, in particular from 5 to 100 bar, preferably 5 to 80 bar and in particular from 10 to 50 bar.

4. Process according to Claims 1 to 3, **characterized in that** the catalyst is used in amounts of 0.2 to 2% by weight and in particular in amounts of 0.5 to 1.0% by weight, in each case based on the amount of fatty alcohol used.

5. Process according to Claims 1 to 4, **characterized in that** the primary alcohols selected are those of the general formula R-OH, in which R is a saturated, linear alkyl radical having 8 to 18, preferably 10 to 16 and in particular 12 to 16, carbon atoms.

6. Process according to one of Claims 1 to 5, **characterized in that** the primary alcohols contain an even number of carbons.

7. Process according to one of Claims 1 to 6, **characterized in that** mixtures of the primary alcohols are used.

8. Process according to one of Claims 1 to 7, **characterized in that** after the reaction the resulting product is purified.

## Revendications

1. Procédé de fabrication d'alcanes saturés linéaires à partir d'alcools primaires dont la chaîne C contient un atome de carbone de plus que l'alcane, par déshydroxyméthylation réductrice des alcools primaires en présence d'hydrogène et d'un catalyseur contenant du nickel à des températures de 100 à 300 °C et des pressions de 2 à 300 bar, l'eau formée pendant la réaction étant éliminée, **caractérisé en ce que** des alcools gras de 8 à 24 atomes de carbone sont choisis en tant qu'alcools primaires et le catalyseur est ajouté en quantités de 0,1 à 3 % en poids.

2. Procédé selon la revendication 1, **caractérisé en ce que** la réaction est réalisée à des températures de 180 à 300 °C, de préférence de 200 à 280 °C, et notamment de 220 à 260 °C.

3. Procédé selon les revendications 1 à 2, **caractérisé en ce que** le procédé est réalisé à des pressions de 2 à 250 bar, notamment de 5 à 100 bar, de préférence de 5 à 80 bar et notamment de 10 à 50 bar.

4. Procédé selon les revendications 1 à 3, **caractérisé en ce que** le catalyseur est utilisé en quantités de 0,2 à 2 % en poids et notamment en quantités de 0,5 à 1,0 % en poids, à chaque fois par rapport à la quantité d'alcool gras utilisé.

5. Procédé selon les revendications 1 à 4, **caractérisé en ce que** des alcools primaires de formule générale R-OH sont choisis en tant qu'alcools primaires, R représentant un radical alkyle linéaire saturé de 8 à 18, de préférence 10 à 16, et notamment 12 à 16 atomes de carbone.

6. Procédé selon l'une quelconque des revendications 1 à 5, **caractérisé en ce que** les alcools primaires contiennent un nombre de carbones pair.

7. Procédé selon l'une quelconque des revendications 1 à 6, **caractérisé en ce que** des mélanges des alcools primaires sont utilisés.

8. Procédé selon l'une quelconque des revendications 1 à 7, **caractérisé en ce que** le produit obtenu est purifié après la réaction.
